**Europäisches Patentamt**

**European Patent Office**

**Office européen des brevets**

(11) Veröffentlichungsnummer: **0 160 895 B1**

(12) **EUROPÄISCHE PATENTSCHRIFT**

(45) Veröffentlichungstag der Patentschrift: **09.09.92**

(21) Anmeldenummer: **85105030.2**

(22) Anmeldetag: **25.04.85**

(51) Int. Cl.⁵: **G01N 33/36**, G01B 11/02

(54) **Verfahren und Anordnung zur kontinuierlichen berührungslosen Messung der Schrumpfung von Textilien.**

(30) Priorität: **08.05.84 DE 3416883**

(43) Veröffentlichungstag der Anmeldung:
**13.11.85 Patentblatt 85/46**

(45) Bekanntmachung des Hinweises auf die Patenterteilung:
**09.09.92 Patentblatt 92/37**

(84) Benannte Vertragsstaaten:
**AT BE CH DE FR GB IT LI LU NL SE**

(56) Entgegenhaltungen:
**WO-A-83/02665**
**DE-A- 3 026 194**
**US-A- 3 721 809**

**TEXTILE RESEARCH JOURNAL, February 1952; pp. 84-86**

(73) Patentinhaber: **Massen, Robert, Prof. Dr.**
**Kämpfenstrasse 39**
**W-7760 Radolfzell 18(DE)**

(72) Erfinder: **Massen, Robert, Prof. Dr.**
**Kämpfenstrasse 39**
**W-7760 Radolfzell 18(DE)**

(74) Vertreter: **Dipl.-Phys.Dr. Manitz Dipl.-Ing., Dipl.-W.-Ing. Finsterwald Dipl.-Ing. Grämkow Dipl.-Chem.Dr. Heyn Dipl.-Phys. Rotermund Morgan, B.Sc.(Phys.) Robert-Koch-Strasse 1 W-8000 München 22(DE)**

**Beschreibung**

Die Erfindung betrifft ein Verfahren zur kontinuierlichen berührungslosen Messung der Schrumpfung von Textilien während des Herstellungsprozesses und eine Anordnung zur Durchführung des Verfahrens.

Beim Weben und Stricken von Garnen wird unerwünschte potentielle Energie im Material gespeichert. Durch späteres Waschen und Trocknen wird diese Energie freigesetzt, so daß sie zur Formerhaltung fehlt: das Material schrumpft. Meistens ist eine Verkürzung in Längsrichtung mit einer Ausweitung in Querrichtung gekoppelt. Beide Vorgänge bezeichnet man als "Schrumpfen".

Die Hersteller unterziehen die von ihnen gefertigten Textilien bei der Fabrikation oft einem bewußten oder kontrollierten Schrumpfprozeß, um spätere unerwünschte Längenänderungen zu verringern. Dabei wird das Material in besonderen Schrumpfmaschinen thermisch und mechanisch bearbeitet, um möglichst viel der eingebrachten potentiellen Energie wieder freizusetzen.

Zur genauen Führung dieses Prozesses wäre es notwendig, die erreichte Schrumpfung in Echtzeit meßtechnisch genau nachzuweisen, und zwar möglichst ohne die oft empfindliche Ware zu berühren. Bei Textilbahnen großer Länge, die während des Herstellungsprozesses in kontinuierlicher Bewegung sind, ist eine direkte Messung der durch die Schrumpfung bewirkten Längenänderung ohne das (unerwünschte) Aufbringen von Marken nicht möglich. Dies gilt insbesondere für die viel benutzte Rundstrickware, die in endlosen Schläuchen anfällt. Viele Hersteller benügen sich daher, von Zeit zu Zeit die Veränderung der Breite der Textilbahn oder des Schlauches manuell nachzumessen und anhand empirischer Zusammenhänge aus der Querausweitung auf die Längenverkürzung zu schließen.

Die Messung des Schrumpfens besteht dabei in der manuellen Auszählung von Maschen entlang einer vorgegebenen Strecke vor und nach dem Schrumpfprozeß (Textile Research Journal, Febr. 1952, Seiten 84 - 86).

Dieses Verfahren ist aus mehreren Gründen in hohem Maße unbefriedigend. Die Messungen sind mit vielen subjektiven Fehlern behaftet und diskontinuierlich. Von dem zweidimensionalen Schrumpfprozeß wird lediglich die Querausweitung erfaßt, während die eigentlich viel wichtigere Längsverkürzung nur indirekt aus einem unterstellten konstanten Zusammenhang ermittelt wird. Das Fehlen genauer Schrumpfungsdaten steht somit einer optimalen und zuverlässigen Führung des Schrumpfprozesses entgegen, was auch erhebliche wirtschaftliche Verluste bedeutet.

Aufgabe der Erfindung ist die Schaffung eines Verfahrens, mit welchem die Schrumpfung von Textilien während des Herstellungsprozesses fortlaufend mit großer Genauigkeit gemessen werden kann, sowie eine Anordnung zur Durchführung des Verfahrens.

Zur Lösung dieser Aufgabe ist das Verfahren nach der Erfindung dadurch gekennzeichnet, daß mit Hilfe eines Bild/Signal-Wandlers das Bild eines Ausschnitts der Textiloberfläche jeweils vor und nach der Schrumpfung aufgenommen und in analoge elektrische Bildsignale umgewandelt wird, daß die analogen Bildsignale bildpunktweise digitalisiert und abgespeichert werden, daß aus den gespeicherten digitalen Bildsignalen die Periode der Textilstruktur in wenigstens einer Richtung vor und nach der Schrumpfung abgeleitet wird, und daß die durch die Schrumpfung bewirkte Längenänderung der Textilware in der Auswertungsrichtung aus dem Verhältnis der beiden in dieser Richtung ermittelten Perioden berechnet wird.

Bei dem Verfahren nach der Erfindung wird die Tatsache ausgenutzt, daß gestrickte, gewirkte und gewebte Textilien eine periodische Struktur aufweisen, deren Periode durch einen Schrumpfprozeß in gleicher Weise beeinflußt wird wie die Abmessung der Textilware. Bei Wirk- und Strickwaren ist die Periode durch die Maschenstruktur gegeben, bei Webwaren durch den Abstand der Schußfäden bzw. der Kettfäden. Das Verhältnis der vor und nach der Schrumpfung in einer bestimmten Richtung gemessenen Perioden ist daher gleich der durch die Schrumpfung bewirkten Längenänderung in der gleichen Richtung.

Mit dem erfindungsgemäßen Verfahren können die Perioden der Textilstruktur in jeder beliebigen Richtung und auch in mehreren Richtungen gleichzeitig erhalten werden. Es ist daher insbesondere möglich, gleichzeitig eine Verkürzung in der Längsrichtung und eine Ausweitung in der Querrichtung zu messen. Die Messung erfolgt auf optischem Weg, ohne daß eine Berührung der Textilware oder ein Eingriff in den Fertigungsprozeß erforderlich ist. Das Verfahren eignet sich daher insbesondere für die Messung der Schrumpfung von kontinuierlich bewegten Textilbahnen oder Schläuchen. Durch geeignete Wahl des Abbildungsmaßstabs kann bei sehr unterschiedlichen Textilien jeweils ein wenige Perioden der Textilstruktur umfassender Bildausschnitt aufgenommen werden, dessen Bildsignale sich für die Ermittlung der mittleren Grundperiode oder eines Vielfachen davon eignen. Von besonderem Vorteil ist die Tatsache, daß es nicht erforderlich ist, die absolute Größe der Periode zu messen; es kommt nur auf das Verhältnis der Perioden vor und nach der Schrumpfung an.

Es gibt mehrere Möglichkeiten, die Periode der Textilstruktur aus den gespeicherten digitalen Bildsignalen abzuleiten. Eine geeignete Maßnahme ist die Bildung der eindimensionalen Autokorrelationsfunktion der digitalen Bildsignale, die von einer in der gewünschten Auswertungsrichtung liegenden Bildpunktreihe stammen. Anstelle der Autokorrelationsfunktion kann auch eine andere verwandte Ähnlichkeitsfunktion gebildet werden. Diese und weitere vorteilhafte Ausgestaltungen des erfindungsgemäßen Verfahrens werden in der folgenden Beschreibung erläutert und sind in den Unteransprüchen gekennzeichnet.

Eine Anordnung zur Durchführung des erfindungsgemäßen Verfahrens enthält nach der Erfindung vorzugsweise einen Bild/Signal-Wandler, der das Bild eines Ausschnitts der Textiloberfläche vor der Schrumpfung aufnimmt und in ein analoges elektrisches Bildsignal umwandelt, einen Bild/Signal-Wandler, der das Bild eines Ausschnitts der Textiloberfläche nach der Schrumpfung aufnimmt und in ein analoges elektrisches Bildsignal umwandelt, einen jedem Bild/Signal-Wandler nachgeschalteten Analog/Digital-Umsetzer zur Digitalisierung der analogen Bildsignale, wenigstens einen Bildsignalspeicher zur Speicherung der von den Analog/Digital-Umsetzern gelieferten digitalen Bildsignale und eine Recheneinheit zur Ableitung der Periode der Textilstruktur aus den gespeicherten digitalen Bildsignalen.

Grundsätzlich eignet sich für die Erfindung jeder Bild/Signal-Wandler, der ein optisches Bild in analoge elektrische Bildsignale umwandeln kann, die sich für eine anschließende bildpunktweise Digitalisierung eignen. Das bekannteste Beispiel für einen solchen Bild/Signal-Wandler ist eine Fernsehkamera, die den erfaßten Bildausschnitt in einem genormten Zeilenraster abtastet. Für die Zwecke der Erfindung ist aber eine solche Fernsehabtastung nicht erforderlich; als Bild/Signal-Wandler können daher auch Matrix- oder Zeilensensoren verwendet werden, wie sie im Handel erhältlich sind.

Weitere Merkmale und Vorteile der Erfindung ergeben sich aus der folgenden Beschreibung von Ausführungsbeispielen anhand der Zeichnung. In der Zeichnung zeigt:

Fig. 1        das Schema einer Anordnung zur Messung der Schrumpfung einer bewegten Textilbahn,
Fig. 2        das Maschenbild einer die Textilbahn bildenden Strickware,
Fig. 3        das digitale Maschenbild und die zugehörigen Helligkeitsprofile in der Quer- und Längsrichtung vor der Schrumpfung,
Fig. 4        das digitalisierte Maschenbild und die zugehörigen Helligkeitsprofile in der Quer- und Längsrichtung nach der Schrumpfung,
Fig. 5        die Autokorrelationsfunktion eines gestörten periodischen Signals,
Fig. 6        die schematische Darstellung einer Bildpunktreihe,
Fig. 7        Diagramme zur Erläuterung eines Verfahrens zur Bildung der Autokorrelationsfunktion aus den digitalen Bildsignalen einer Bildpunktreihe,
Fig. 8        die gemäß dem Verfahren von Fig. 7 erhaltene Autokorrelationsfunktion,
Fig. 9        die an einer Strickware in der Längsrichtung vor und nach dem Schrumpfen gewonnenen Autokorrelationsfunktionen,
Fig. 10       die an einer Strickware in der Querrichtung vor und nach dem Schrumpfen gewonnenen Autokorrelationsfunktionen,
Fig. 11       Diagramme zur Erläuterung eines anderen Verfahrens zur Bildung der Autokorrelationsfunktion aus den digitalen Bildsignalen einer Bildpunktreihe,
Fig. 12       die gemäß dem Verfahren von Fig. 11 erhaltene Autokorrelationsfunktion,
Fig. 13       ein Diagramm des Verhältnisses der Streuungen der nach den Verfahren von Fig. 7 und 11 erhaltenen Meßergebnisse,
Fig. 14       das Diagramm einer anderen, zur Bestimmung der Periode geeigneten Ähnlichkeitsfunktion,
Fig. 15       ein Diagramm zur Erläuterung der Gewinnung mehrerer Perioden aus der Autokorrelationsfunktion von Fig. 12,
Fig. 16       ein Diagramm zur Erläuterung der Auswertung einer Autokorrelationsfunktion,
Fig. 17       das Maschenbild einer Strickware zur Erläuterung der Auswahl geeigneter Bildpunktreihen,
Fig. 18       das Helligkeitsprofil des Maschenbildes von Fig. 17 in der Querrichtung,
Fig. 19       das Maschenbild einer Strickware mit schräglaufenden Maschenreihen,
Fig. 20       eine zur Ermittlung des Schräglaufs aus dem Maschenbild von Fig. 19 gebildete Kreuzkorrelationsfunktion und
Fig. 21       das Diagramm einer zur Ermittlung der Periode der Textilstruktur geeigneten spektralen Transformation des Maschenbildes.

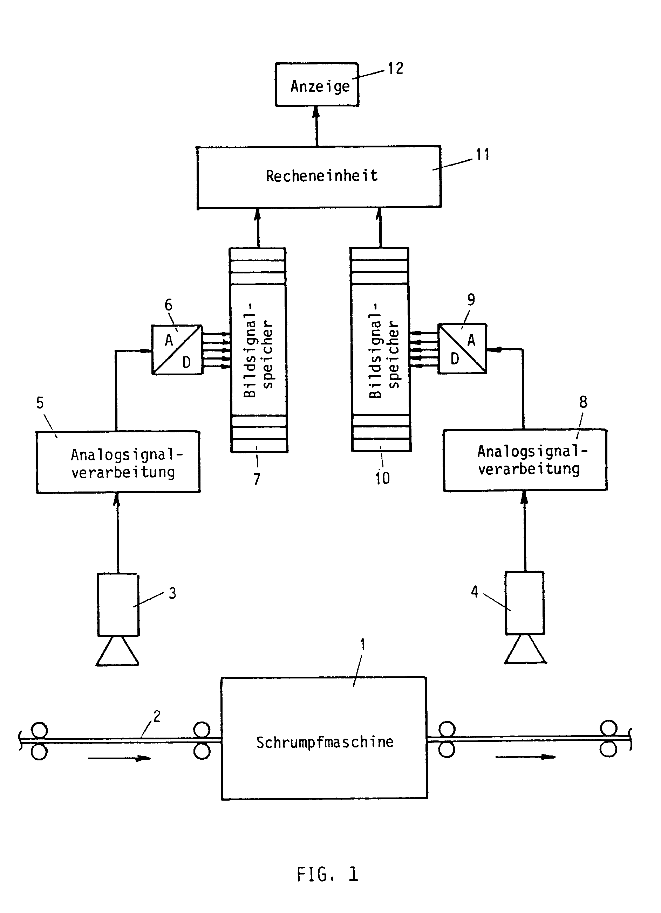

Fig. 1 zeigt eine Schrumpfmaschine 1, durch die eine Textilbahn 2 in der Richtung der Pfeile hindurchgeführt wird. Die Textilbahn 2 kann ein Gewebe oder eine Maschenware (Wirk- oder Strickware) sein. Als Beispiel wird in der folgenden Beschreibung angenommen, daß es sich bei der Textilbahn 2 um eine Strickware handelt.

Die Textilbahn 2 wird in der Schrumpfmaschine 1 thermisch und mechanisch bearbeitet, damit eine bewußte und kontrollierte Schrumpfung eintritt, die später unerwünschte Längenänderungen durch Schrumpfung weitgehend verhindert. Der Grad der Schrumpfung kann durch die Einstellung verschiedener Beeinflussungsgrößen an der Schrumpfmaschine 1 gesteuert werden.

Zur genauen Führung des Schrumpfprozesses ist es notwendig, die erreichte Schrumpfung meßtechnisch genau nachzumessen. Zu diesem Zweck ist am Eingang der Schrumpfmaschine 1 eine Fernsehkamera 3 angeordnet, die einen Bildausschnitt der vorbeilaufenden Textilbahn 2 vor dem Eintritt in die Schrumpfmaschine 1 aufnimmt. Am Ausgang der Schrumpfmaschine 1 ist eine zweite Fernsehkamera 4 angeordnet, die einen ähnlichen Bildausschnitt der aus der Schrumpfmaschine 1 austretenden Textilbahn 2 aufnimmt.

Jede Fernsehkamera 3 und 4 ist ein Bild/Signal-Wandler, der den aufgenommenen Bildausschnitt in ein analoges elektrisches Videosignal umwandelt, das am Ausgang der Fernsehkamera abgegeben wird. Das von der Fernsehkamera 3 abgegebene analoge Videosignal wird nach der erforderlichen analogen Aufbereitung in einer Analogsignalverarbeitungsschaltung 5 einem Analog-Digital-Umsetzer 6 zugeführt, in dem es digitalisiert wird. Der Analog-Digital-Umsetzer 6 entnimmt aus dem zugeführten Analogsignal periodisch Abtastwerte und setzt jeden Abtastwert in ein digitales Signal um. Jeder Abtastwert entspricht einem Bildpunkt (Pixel) auf einer Abtastzeile des Fernsehbildes, und seine Amplitude entspricht dem Helligkeitswert des Bildpunktes. Im Analog-Digital-Umsetzer 6 wird der abgetastete Amplitudenwert quantisiert und beispielsweise in eine binäre Codegruppe umgesetzt, die den Helligkeitswert in Form einer Binärzahl darstellt. Die Stellenzahl der Binärzahl, also die Bitzahl der Codegruppe, hängt von der gewünschten Quantisierungsauflösung ab. Im einfachsten Fall kann eine Quantisierung mit einem Bit genügen, d.h. alle unter einem vorgegebenen Schwellenwert liegenden Helligkeitswerte werden durch den Binärwert "0" (schwarz) und alle über dem Schwellenwert liegenden Helligkeitswerte werden durch den Binärwert "1" (weiß) wiedergegeben.

Die digitalen Ausgangssignale des Analog-Digital-Umsetzers 6 werden in einen schnellen Bildsignalspeicher 7 eingegeben, dessen Kapazität so bemessen ist, daß er die von allen Bildpunkten eines vollständigen Abtastrasters der Fernsehkamera 3 stammenden digitalen Signale speichern kann.

In gleicher Weise wird das von der Fernsehkamera 4 abgegebene analoge Videosignal nach analoger Aufbereitung in einer Analogsignalverarbeitungsschaltung 8 einem Analog-Digital-Umsetzer 9 zugeführt, in dem es digitalisiert wird. Die digitalen Ausgangssignale des Analog-Digital-Umsetzers 9 werden in einem schnellen Bildsignalspeicher 10 abgespeichert. Die Schaltungsbestandteile 8, 9 und 10 haben den gleichen Aufbau und die gleiche Funktionsweise wie die Schaltungsbestandteile 5, 6 bzw. 7.

Mit den Lese-Ausgängen der beiden Bildsignalspeicher 7 und 10 ist eine Recheneinheit 11 verbunden, die beispielsweise ein Mikrocomputer ist. Die Recheneinheit 11 kann die in den Bildsignalspeichern 7 und 10 gespeicherten digitalen Bildsignale abrufen. Sie bestimmt daraus nach einem der später noch erläuterten Verfahren die Schrumpfung der Textilbahn 2 in der Längsrichtung und in der Querrichtung. Die ermittelte normierte Schrumpfung kann beispielsweise mittels einer digitalen Anzeigevorrichtung 12 angezeigt werden, damit eine Bedienungsperson die Schrumpfmaschine 1 entsprechend einstellen kann. Es ist natürlich auch möglich, die von der Recheneinheit 11 ermittelten Schrumpfungswerte unmittelbar zur Regelung des Schrumpfprozesses zu verwenden.

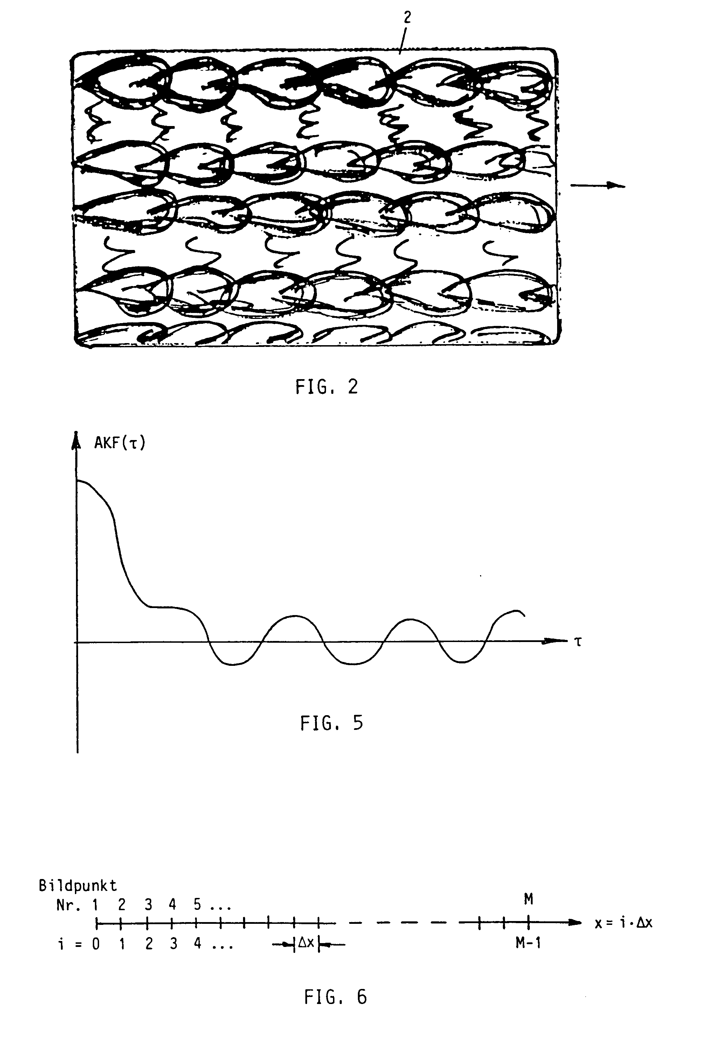

Fig. 2 zeigt in schematisierter Form den von einer Fernsehkamera 3 bzw. 4 erfaßten Bildausschnitt der Textilbahn 2, wenn diese eine Strickware ist. Man erkennt in Fig. 2 die Maschenstruktur der Strickware, wobei die Längsrichtung der Strickware der durch einen Pfeil angedeuteten Bewegungsrichtung entspricht. Die Schrumpfung der Strickware ist ein globaler Längenänderungsprozeß mit einer Verkürzung in Längsrichtung und einer Ausweitung in Querrichtung. Diese geometrischen Veränderungen sind auch an der einzelnen Masche nachweisbar.

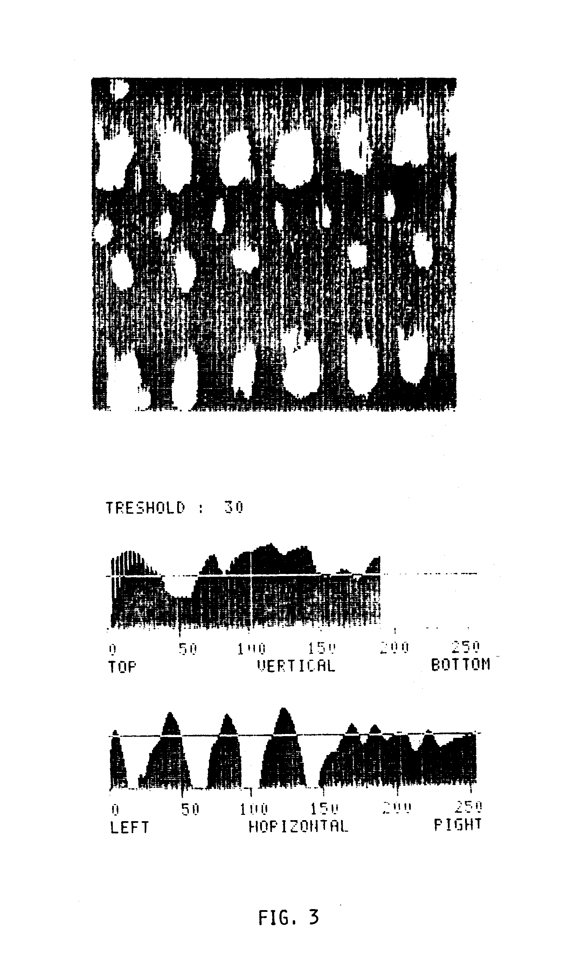

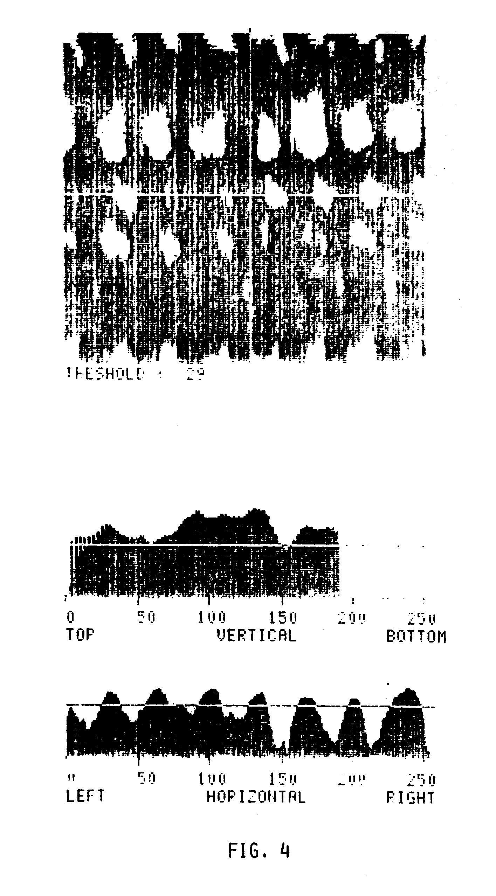

Fig. 3 zeigt oben ein binarisiertes, also durch 1 Bit-Quantisierung in Schwarz-Weiß-Werte umgesetztes wirkliches Maschenbild, das eine Fernsehkamera vor der Schrumpfung aufgenommen hat, und in Fig. 4 ist oben das entsprechende binarisierte Maschenbild nach der Schrumpfung dargestellt. Das Material ist schwarz und die Maschenöffnungen sind weiß wiedergegeben. Unter jedem binarisierten Maschenbild ist ein zugehöriges Helligkeitsprofil in der Querrichtung (vertikal) und darunter ein zugehöriges Helligkeitsprofil in der Längsrichtung (horizontal) dargestellt. Aus den binarisierten Maschenbildern ist ersichtlich, daß die Maschen und Maschenöffnungen in Form, Größe und Abstand stark schwanken. Es ist daher nicht möglich, durch Auswertung einer einzelnen Masche eindeutige geometrische Maße zur Beurteilung der Schrumpfung abzuleiten. Auch die direkte Auswertung des Helligkeitsprofils gestattet dies nicht, weil auch hier große individuelle Schwankungen auftreten.

Bei dem mit Hilfe der Anordnung von Fig. 1 durchgeführten Verfahren wird ein anderer Weg beschritten. Das Maschenbild von Fig. 2 und die binarisierten Maschenbilder von Fig. 3 und 4 lassen eine gewisse Periodizität der abgebildeten Struktur sowohl in der Längsrichtung als auch in der Querrichtung erkennen. Das angewendete Verfahren beruht auf dem Grundgedanken, die Strickware als einen stark gestörten periodischen Prozeß zu modellieren. Wenn es gelingt, die Grundperiode ausreichend genau aus dem Rauschen zu isolieren, dann lassen sich Längenänderungen direkt als Änderungen dieser Grundperiode angeben.

Die Recheneinheit 11 ermittelt die rauschbefreite Grundperiode sowohl in der Längsrichtung als auch in der Querrichtung vor der Schrumpfung und nach der Schrumpfung aus den in den Bildsignalspeichern 7 und 10 gespeicherten digitalen Signalen durch Anwendung eines geeigneten Rechenverfahrens. Die im Bildsignalspeicher 7 gespeicherten digitalen Signale ergeben die beiden Grundperioden vor dem Schrumpfen, und die im Bildsignalspeicher 10 gespeicherten digitalen Signale ergeben die beiden Grundperioden nach dem Schrumpfen. Aus den Unterschieden der Grundperioden vor und nach dem Schrumpfen wird die normierte Schrumpfung in der Längsrichtung und in der Querrichtung berechnet.

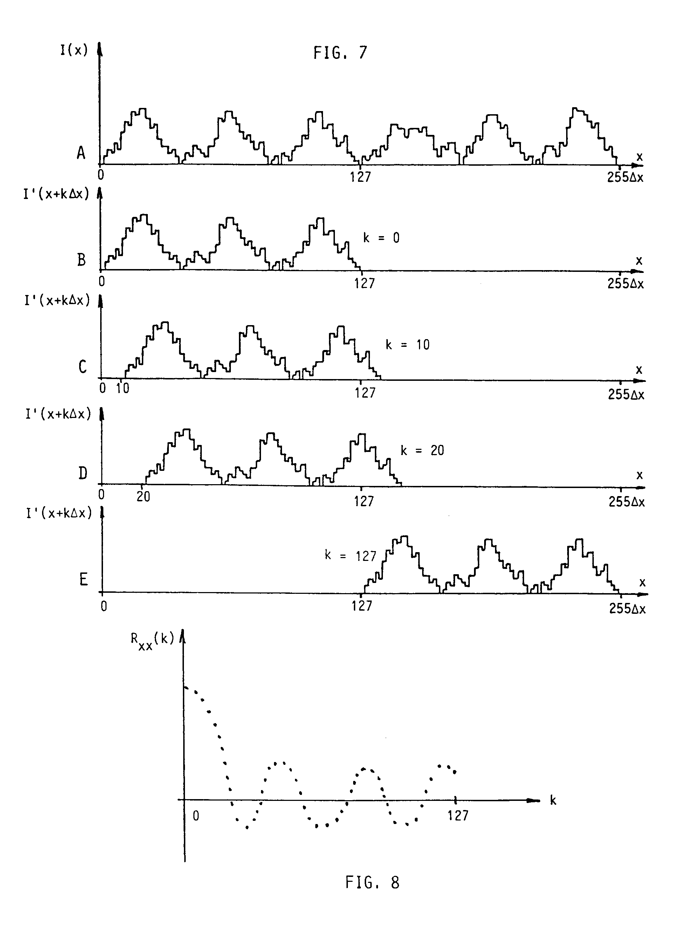

Ein geeignetes Verfahren zur Bestimmung der rauschbefreiten Grundperiode ist die Bildung der eindimensionalen Autokorrelationsfunktion des mit der Fernsehkamera abgetasteten Bildausschnitts in der betreffenden Richtung (Längs- oder Querrichtung). Bekanntlich ist die Autokorrelationsfunktion (abgekürzt: AKF) die Korrelation einer Zeitfunktion $x(t)$ mit der gleichen Zeitfunktion $x(t-\tau)$ als Funktion der Zeitverzögerung $\tau$. Wie Fig. 5 zeigt, besteht die Autokorrelationsfunktion eines gestörten periodischen Signals aus einem Anteil, welcher die Überlagerung von Rauschen und Periode beschreibt, und aus einem Anteil, welcher nur von der Periode abhängt. Zur Bestimmung der Grundperiode genügt es daher, diesen zweiten Teil auszuwerten. Die Grundperiode ergibt sich aus dem Abstand zweier aufeinanderfolgender Maxima oder mit größerer Genauigkeit aus dem Gesamtabstand von N konsekutiven Maxima und Division durch N.

Bei dem hier vorliegenden Fall von Fernsehvideosignalen ist die Zeitfunktion durch die Helligkeitswerte aufeinanderfolgender Bildpunkte bestimmt. Zum besseren Verständnis ist in Fig. 6 schematisch der Anfang und das Ende einer Bildzeile dargestellt, wobei die fortlaufende Koordinate der Zeilenbildpunkte mit x bezeichnet ist. Die durch die periodische Abtastung in den Analog-Digital-Umsetzern 6 und 9 ausgewählten Bildpunkte liegen in gleichmäßigen Abständen $\Delta x$ voneinander. Die ganze Zeile umfaßt M Bildpunkte. Wenn der erste Bildpunkt als Bildpunkt Nr. 1 bezeichnet wird und die Koordinate $x = 0$ hat, so hat der Bildpunkt Nr. $(i+1)$ die Koordinate $i \cdot \Delta x$, und der Bildpunkt Nr. M hat die Koordinate $(M-1) \cdot \Delta x$.

Die Autokorrelationsfunktion ist definiert durch die Summe der paarweisen Produkte der Helligkeitswerte von Bildpunkten einer Zeile mit den Helligkeitswerten von Bildpunkten der gleichen, um k Bildpunkte verschobenen Zeile

$$R_{xx}(k) = \sum_{i=0}^{L-1} I(i \cdot \Delta x) \cdot I((i+k) \cdot \Delta x) \qquad (1)$$

mit

k $\quad= 0, 1, 2, ..., (Q - 1)$

Hierbei ist:

$I(i \cdot \Delta x)$: der Helligkeitswert (Grauwert) des Bildpunktes mit der Koordinate $i \cdot \Delta x$;

L: die Anzahl der für einen Wert des Verschiebeoperators k verarbeiteten Bildpunktpaare;

Q: die Anzahl der berechneten Stützwerte der Autokorrelationsfunktion.

Für jeden Wert des Verschiebeoperators k erhält man nach der Gleichung (1) einen Stützwert der Autokorrelationsfunktion, insgesamt also Q Stützwerte. Für bestimmte Werte von k nehmen die Stützwerte ein Maximum an.

Im Diagramm A von Fig. 7 ist schematisch die digitalisierte Helligkeitsfunktion I(x) einer Bildzeile mit M = 256 Bildpunkten dargestellt, wie sie in einem der Bildsignalspeicher 7, 10 von Fig. 1 abgespeichert ist. Zur Bildung der Autokorrelationsfunktion wird die Helligkeitsfunktion I(x) mit einem halb so langen Auszug I'(x + k·Δx) verglichen, der nacheinander um k = 0, 1, 2, ..., 127 Bildpunkte verschoben wird. In den Diagrammen B, C, D und E von Fig. 7 ist der gleiche Auszug I'(x + k·Δx) nach einer Verschiebung um k = 0, k = 10, k = 20 bzw. k = 127 dargestellt. Für jede Verschiebung wird jeder digitalisierte Helligkeitswert des Auszugs I'(x + k·Δx) mit dem vertikal darüberstehenden Helligkeitswert der Helligkeitsfunktion I(x) des Diagramms A multipliziert. Die Anzahl L der für jeden Wert von k nach der Gleichung (1) verarbeiteten Bildpunktpaare beträgt also L = M/2 = 128. Damit für alle Werte von k noch L = M/2 Vergleichspaare vorhanden sind, muß auch Q auf M/2 begrenzt bleiben. Somit ergibt sich beispielsweise bei einer Bildzeile mit M = 256 Bildpunkten die im Diagramm von Fig. 8 dargestellte Autokorrelationsfunktion mit Q = 128 Stützwerten. Die Autokorrelationsfunktion ist nicht kontinuierlich, sondern punktweise aufgebaut, wobei jeder Punkt einem Stützwert entspricht. Für bestimmte Werte von k haben die Stützwerte Maxima, weil sich bei den entsprechenden gegenseitigen Verschiebungen k·Δx infolge der Grundperiode der verglichenen

5

Strukturen die maximale Übereinstimmung ergibt.

Für das an Hand von Fig. 7 und 8 erläuterte Verfahren zur Bildung der Autokorrelationsfunktion lautet die Gleichung (1) mit den angegebenen Werten L = M/2 und Q = M/2:

$$R_{xx}(k) = \sum_{i=0}^{M/2-1} I(i \cdot \Delta x) \cdot I'((i+k) \cdot \Delta x) \qquad (2)$$

mit

k = 0, 1, 2, ..., (M/2-1).

Die Bildung der Autokorrelationsfunktion wird in der Anordnung von Fig. 1 durch die Abspeicherung der digitalisierten Helligkeitswerte in den Bildsignalspeichern 7, 10 sehr erleichtert, weil die Recheneinheit 11 aus den Bildsignalspeichern die den gegeneinander versetzten Zeilenabschnitten entsprechenden Datensätze wiederholt auslesen kann.

Zur Vergrößerung der statistischen Genauigkeit kann man die Autokorrelationsfunktionen von mehreren ausgewählten Bildzeilen summieren. Technische Fernsehabtastsysteme mit anschließender Digitalisierung und Abspeicherung der Grauwerte arbeiten oft mit 256 Bildzeilen.

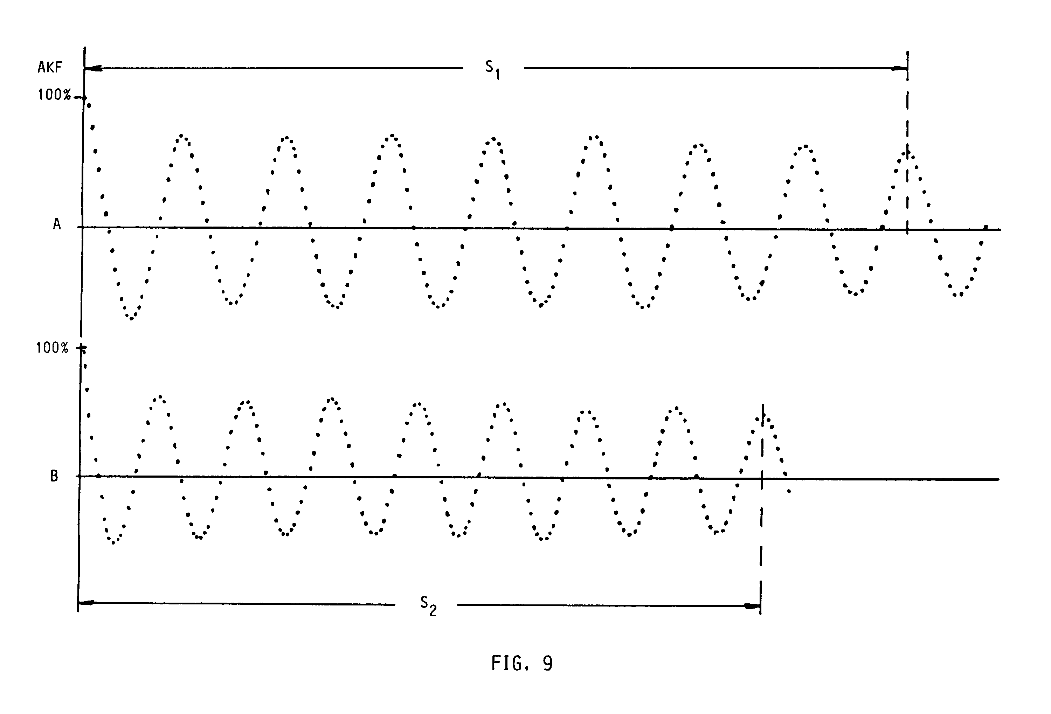

Das Diagramm A von Fig. 9 zeigt eine solche über acht Bildzeilen summierte Autokorrelationsfunktion, die von einer Strickware vor dem Schrumpfen aufgenommen worden ist, und im Diagramm B von Fig. 9 ist die entsprechende Autokorrelationsfunktion der Strickware nach dem Schrumpfen dargestellt. Die Strickware war so ausgerichtet, daß die Zeilenrichtung des Fernsehbildes der Längsrichtung der Strickware entsprach. Der Unterschied in der Grundperiode zwischen der ungeschrumpften und der geschrumpften Strickware läßt sich deutlich erkennen und mit Hilfe der Recheneinheit leicht auswerten. Mit $S_1$ ist die Gesamtperiode von acht Maschen vor dem Schrumpfen und mit $S_2$ die entsprechende Gesamtperiode nach dem Schrumpfen bezeichnet. Die normierte Längsschrumpfung kann dann berechnet werden als:

$$S = \frac{S_1 - S_2}{S_1} \cdot 100\% \qquad (3)$$

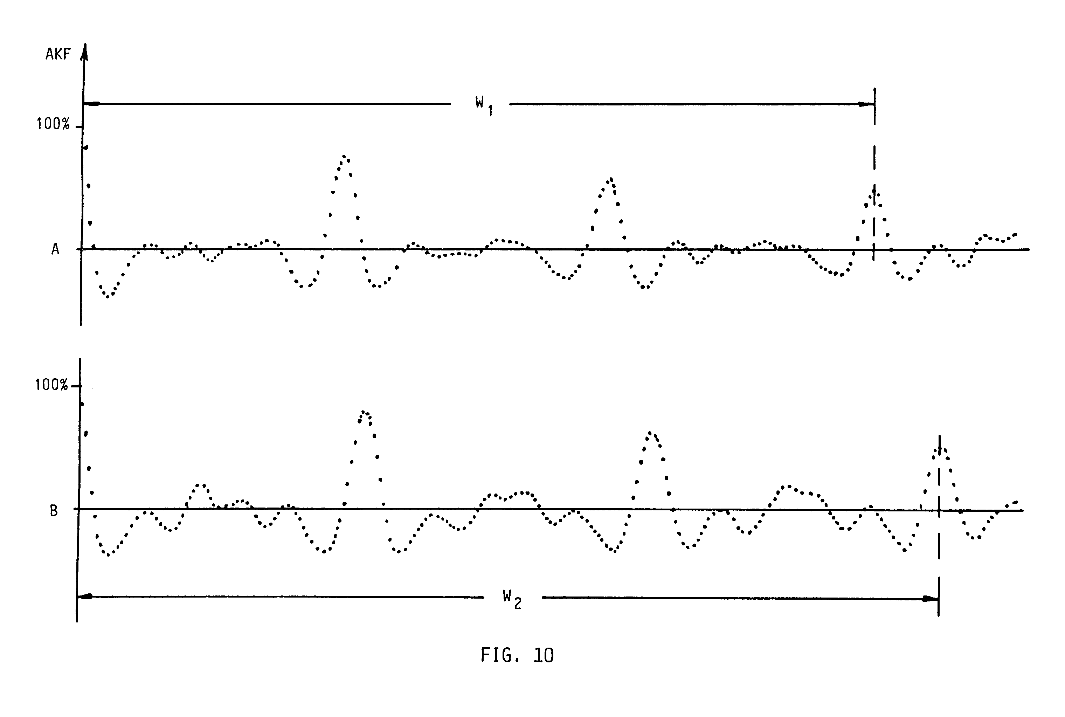

In gleicher Weise lassen sich auch die Längenänderungen in der Querrichtung bestimmen. Dabei werden die im Fernsehbild vertikal übereinanderliegenden Bildpunkte als Bildspalte bezeichnet. Die Autokorrelationsfunktion einer Bildspalte wird in gleicher Weise wie die Autokorrelationsfunktion einer Bildzeile berechnet, wobei der Verschiebeoperator k dem Zeilenabstand entspricht. Das Diagramm A von Fig. 10 zeigt die über acht parallele Bildspalten summierte Autokorrelationsfunktion der Strickware in der Querrichtung vor dem Schrumpfen, und das Diagramm B von Fig. 9 zeigt die entsprechende Autokorrelationsfunktion nach dem Schrumpfen. Mit $W_1$ ist die über drei Grundperioden ermittelte Gesamtperiode vor dem Schrumpfen und mit $W_2$ die entsprechende Gesamtperiode nach dem Schrumpfen bezeichnet. Wie zu erkennen ist, ist die Periode in der Querrichtung vor dem Schrumpfungsprozeß kleiner als nachher. Das Material erfährt eine Querausweitung, und die normierte Querausweitung berechnet sich zu

$$W = \frac{W_2 - W_1}{W_2} \cdot 100\% \qquad (4)$$

In beiden Fällen ist es also nicht erforderlich, die Grundperiode selbst zu ermitteln; die gesuchte Schrumpfung bzw. Ausweitung kann unmittelbar aus den Gesamtperioden berechnet werden, die einem bekannten Vielfachen der Grundperioden entsprechen.

In vielen praktischen Fällen ist es nicht erforderlich, den gesamten Amplitudenverlauf der Bildpunkthelligkeit zu erfassen, da wesentliche Aussagen der Autokorrelationsfunktion auch dann noch erhalten bleiben, wenn nur die auf ein Bit quantisierten Signale korreliert werden (Polaritätskorrelationsfunktion).Hierdurch vereinfacht sich die Berechnung erheblich, da statt Produkten nur noch logische Verknüpfungen zu berechnen sind. Die Meßgenauigkeit kann dadurch erhöht werden, daß das analoge Videosignal vor der Digitalisierung hochpaß-gefiltert wird. Durch eine solche Filterung werden die hochfrequenten Bildstrukturen, wie Kanten, betont und die Korrelationsmaxima verschärft. Dadurch läßt sich die Grundperiode mit besserer Genauigkeit ermitteln. Bei der Anordnung von Fig. 1 sind die erforderlichen Hochpaßfilter dann in den Analogsignalverarbeitungsschaltungen 5 und 8 enthalten.

Vorzugsweise sind die Fernsehkameras so ausgerichtet, daß die Bewegungsrichtung der Textilbahn parallel zur Zeilenabtastrichtung verläuft, da hierdurch die Bewegungsunschärfe minimal wird. Bei großer Vorschubgeschwindigkeit muß die Textilbahn stroposkopisch synchron zum Bildwechsel beleuchtet werden. Hierzu verwendet man vorteilhafterweise einen Infrarot-Blitz und eine infrarotempfindliche Halbleiterkamera.

Da sich die Schrumpfungswerte in den meisten praktischen Fällen nur langsam ändern, kann die Anordnung von Fig. 1 dadurch vereinfacht werden, daß anstelle der beiden schnellen Bildsignalspeicher 7 und 10 nur ein Bildsignalspeicher verwendet wird, in den die Ausgangssignale der Analog-Digital-Umsetzer 6 und 9 abwechselnd eingegeben werden.

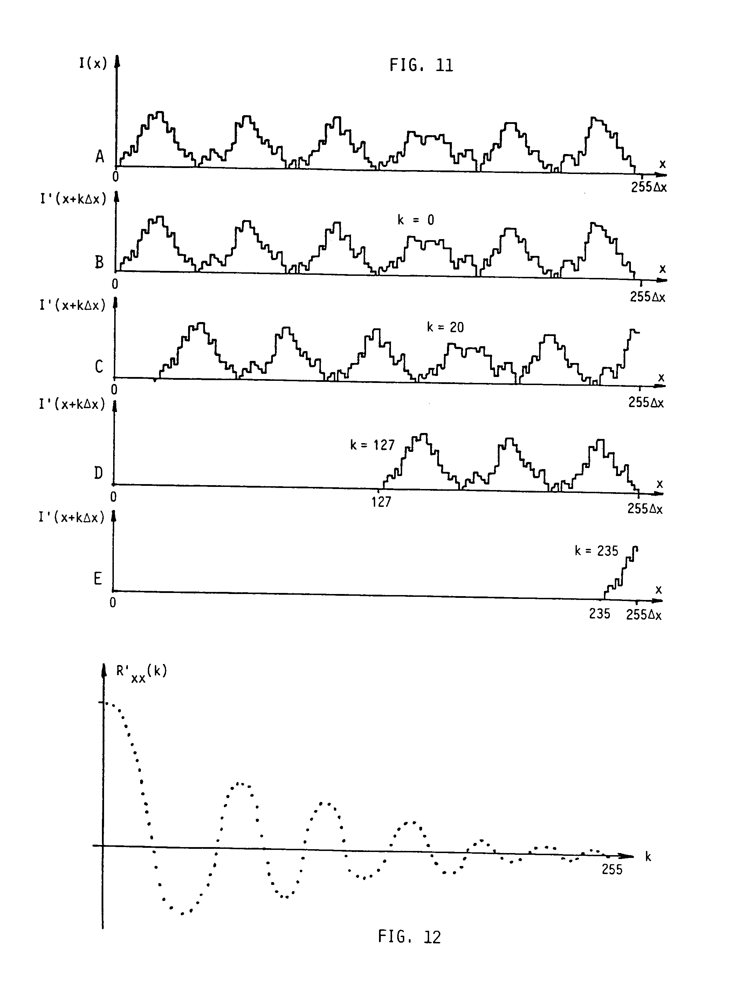

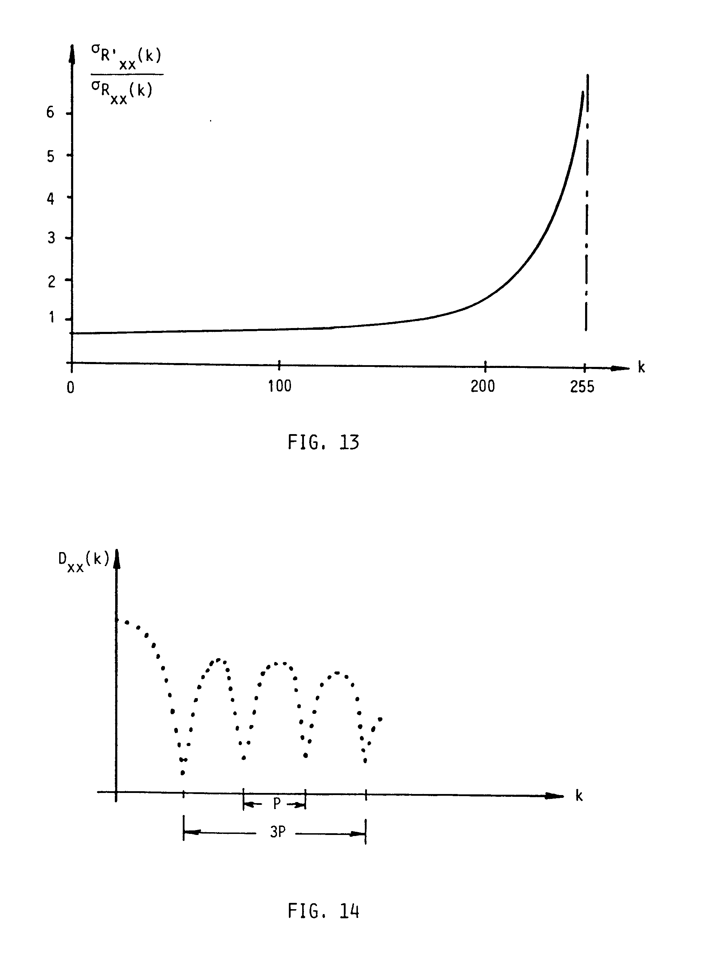

In den Figuren 11 und 12 ist in ähnlicher Form wie in Fig. 7 und 8 ein neues Verfahren zur Bildung der Autokorrelationsfunktion dargestellt. Das Diagramm A von Fig. 11 zeigt wieder schematisch die digitalisierte Helligkeitsfunktion I(x) einer Bildzeile mit M = 256 Bildpunkten, und in den Diagrammen B, C, D und E von Fig. 11 ist der Auszug I'(x + k·Δx), der für verschiedene Werte des Verschiebeoperators k mit der Helligkeitsfunktion I(x) verglichen wird, nach einer Verschiebung um k = 0, k = 20, k = 127bzw. k = 235 dargestellt. Der Unterschied zu dem an Hand von Fig. 7 und 8 erläuterten Verfahren besteht darin, daß der Auszug I'(x + k·Δx) nicht eine konstante Länge von M/2 Bildpunkten hat, sondern jeweils den gesamten Rest der verschobenen Bildzeile umfaßt, der sich mit der nicht verschobenen Bildzeile überlappt. Der Vergleich erfolgt daher bei der Verschiebung k = 0 mit M Bildpunktpaaren und wird mit zunehmender Verschiebung k über eine entsprechend abnehmende Anzahl von L = M-k korrespondierenden Bildpunktpaaren durchgeführt. Die Verschiebung kann theoretisch bis zu dem Wert k = M-1 erfolgen, so daß Q = M Stützwerte der Autokorrelationsfunktion erhalten werden, wie im Diagramm von Fig. 12 dargestellt ist. Die Gleichung (1) lautet für diesen Fall:

$$R'_{xx}(k) = \sum_{i=0}^{M-k-1} I(i \cdot \Delta x) \cdot I'((i+k) \cdot \Delta x) \qquad (5)$$

mit

$k \quad = 0, 1, 2, ..., (M-1);$

$L \quad = M-k; \ Q = M.$

Bis zur Verschiebung k = M/2 werden die Produkte von mehr Paaren aufsummiert als bei dem Verfahren von Fig. 7, so daß in diesem Bereich eine bessere statistische Meßgenauigkeit erzielt wird. Bei höheren Werten von k nimmt die Meßunsicherheit zu, was sich im Diagramm von Fig. 12 darin äußert, daß die Maxima immer weniger ausgeprägt werden.

Bei dem an Hand von Fig. 7 und 8 erläuterten Verfahren nach Gleichung (2) erhält man eine größte Standardabweichung

$$\sigma_{R_{xx}}(k) = \frac{1}{\sqrt{M/2}} \neq f(k), \qquad (6)$$

die unabhängig von der Verschiebung k ist, wohingegen bei dem an Hand von Fig. 11 und 12 erläuterten Verfahren nach Gleichung (5) die Standardabweichung eine Funktion der Verschiebung k ist:

$$\sigma_{R'_{xx}}(k) = \frac{1}{\sqrt{M-k}} = f(k) \qquad (7)$$

Setzt man die beiden Streuungen ins Verhältnis zueinander, so erhält man

$$\frac{\sigma_{R'_{xx}(k)}}{\sigma_{R_{xx}(k)}} = \frac{0,7}{\sqrt{1-k/M}} \tag{8}$$

Fig. 13 zeigt diesen Verlauf, aus welchem man entnehmen kann, daß die statistische Ungenauigkeit beim zweiten Verfahren gegenüber dem ersten Verfahren mit zunehmender Verschiebung k zunächst nur sehr langsam ansteigt. Man kann daher bei einem Datensatz von M Bildpunkten praktisch eine Ähnlichkeitsfunktion von etwa 0,9•M Stützwerten nach dem zweiten Verfahren berechnen, ohne daß die gegenüber dem ersten Verfahren zusätzlich gewonnenen Stützwerte unzulässig streuen. Somit wird durch das zweite Verfahren der Meßbereich nahezu verdoppelt, ohne daß die Anzahl der aufzunehmenden und abzuspeichernden Bildpunkte vergrößert werden muß.

Anstelle der Autokorrelationsfunktion können auch andere Ähnlichkeitsfunktionen zur Ermittlung der mittleren Grundperiode des Maschenbildes verwendet werden. Da zur Berechnung der Autokorrelationsfunktion Produkte der gespeicherten Helligkeitswerte gebildet werden müssen, ist ein erheblicher Zeitaufwand und Hardware-Aufwand erforderlich. Eine einfacher zu berechnende Ähnlichkeitsfunktion ist die Summe der Betragsdifferenzen der gegeneinander verschobenen Helligkeitswerte:

$$D_{xx}(k) = \sum_{i=0}^{L-1} \left| I(i \cdot \Delta x) - I((i+k) \cdot \Delta x) \right| \tag{9}$$

mit

$k = 0, 1, 2, ..., (Q-1)$

Dabei haben die verschiedenen Buchstaben die zuvor bei der Gleichung (1) angegebene Bedeutung. Die Berechnung kann beispielsweise nach dem Verfahren von Fig. 7 mit $L = M/2$ und $Q = M/2$ Stützwerten oder auch nach dem Verfahren von Fig. 11 mit $L = M-k$ und maximal $Q = M$ Stützwerten erfolgen.

Die Ähnlichkeitsfunktion $D_{xx}(k)$ liefert prinzipiell die gleichen Aussagen wie die Autokorrelationsfunktion $R_{xx}(k)$, kann aber mit weitaus weniger Aufwand ermittelt werden. Fig. 14 zeigt den typischen Verlauf der Ähnlichkeitsfunktion $D_{xx}(k)$ für ein Maschenbild. Die mittlere Grundperiode P bzw. die für die Berechnung der Schrumpfung vorzugsweise verwendete Gesamtperiode mehrerer Maschen läßt sich am genauesten aus den periodischen Minima bestimmen, da diese schärfer ausgeprägt sind als die Maxima.

Weitere geeignete Ähnlichkeitsfunktionen, die zur Ermittlung der Periode des Maschenbildes aus den gespeicherten digitalisierten Helligkeitswerten verwendet werden können, sind aus der Literatur bekannt.

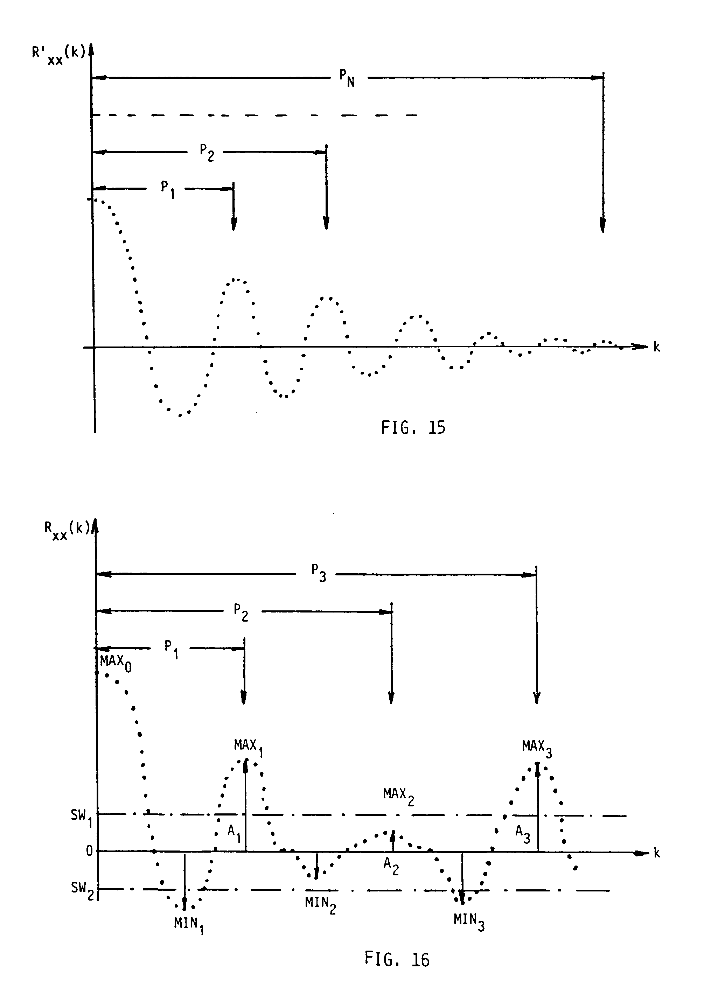

Eine Erhöhung der statistischen Meßgenauigkeit kann durch die Bildung gewichteter Mittelwerte mehrerer aus einer Ähnlichkeitsfunktion gewonnener Perioden erzielt werden. Beispielsweise können bei der nach dem Verfahren von Fig. 11 und 12 gewonnenen Autokorrelationsfunktion $R'_{xx}(k)$, die in Fig. 15 nochmals dargestellt ist, die vom Ursprung aus gemessenen Perioden $P_1$, $P_2$, ..., $P_N$ der aufeinanderfolgenden Maxima folgendermaßen zu einer mittleren Gesamtperiode $\overline{P}$ verknüpft werden:

$$\overline{P} = \frac{a_1 P_1 + a_2 P_2 + ... + a_N P_N}{a_1 + a_2 + ... + a_N} \tag{10}$$

Dadurch wird eine Auflösung erreicht, die größer als der Quantisierungsschritt 1/M ist. Dabei werden die Gewichtungsfaktoren vorzugsweise so gewählt, daß sie gegenläufig zur Zunahme der Streuung nach Gleichung (8) verlaufen, so daß stärker streuende Meßwerte schwächer in die Mittelung eingehen als weniger stark streuende.

Eine weitere Verbesserung der statistischen Meßgenauigkeit kann dadurch erzielt werden, daß die Perioden mehrerer Ähnlichkeitsfunktionen aus mehreren Bildzeilen desselben Maschenbildes oder mehrerer aufeinanderfolgender Maschenbilder gemittelt werden und daß die Ähnlichkeitsfunktion vor der Bestimmung der Perioden numerisch geglättet und interpoliert wird.

Durch gröbere Unregelmäßigkeiten in der Maschenstruktur, z.B. infolge von erheblichen Ungleichmäßigkeiten der Maschenwerte, infolge von Falten in der Textilbahn usw., kann es vorkommen, daß auch in der periodischen Ähnlichkeitsfunktion Unregelmäßigkeiten auftreten, die zu Fehlern bei der Ermittlung der Grundperiode bzw. der gemittelten Gesamtperiode führen können. Fig. 16 zeigt als Beispiel eine Autokorrelationsfunktion $R_{xx}(k)$ mit vier aufeinanderfolgenden Maxima $MAX_0$, $MAX_1$, $MAX_2$, $MAX_3$, wobei das Maximum $MAX_2$ infolge von Unregelmäßigkeiten im Maschenbild nur sehr schwach ausgeprägt ist. Zur Vermeidung von Fehlern ist es vorgesehen, bei der Ermittlung der Grundperiode oder der mittleren Gesamtperiode aus der Ähnlichkeitsfunktion nur solche Ausschnitte zu verwenden, die ein bestimmtes Gütekriterium erfüllen. Ein solches Kriterium kann typischerweise die Amplitude sein. Ein Maximum wird nur dann zur Auswertung herangezogen, wenn seine Amplitude einen vorgegebenen Schwellenwert $SW_1$ überschreitet. Wenn beispielsweise aus der Autokorrelationsfunktion $R_{xx}(k)$ von Fig. 16 die mittlere Gesamtperiode nach der obigen Gleichung (10) ermittelt wird, werden durch die Maxima $MAX_1$ und $MAX_3$ bestimmte Perioden $P_1$ und $P_3$ für die Mittelung verwertet, weil die Amplituden $A_1$ imd $A_3$ dieser Maxima den Schwellenwert $SW_1$ überschreiten. Dagegen wird die durch das Maximum $MAX_2$ bestimmte Periode $P_2$ nicht verwertet, weil die Amplitude $A_2$ den Schwellenwert $SW_1$ nicht erreicht und daher das Maximum $MAX_2$ als nicht sicher genug ausgeprägt erscheint.

Wenn die Ermittlung der Periode des Maschenbildes aufgrund der Minima erfolgt, werden in entsprechender Weise nur solche Minima zur Auswertung herangezogen, deren Amplitude einen vorgegebenen Schwellenwert $SW_2$ unterschreitet. Somit würden bei der Autokorrelationsfunktion $R_{xx}(k)$ von Fig. 16 nur die Minima $MIN_1$ und $MIN_3$ ausgewertet, nicht jedoch das Minimum $MIN_2$. Wie zuvor an Hand von Fig. 14 erläutert wurde, kommt die Auswertung der Minima insbesondere dann in Betracht, wenn als Ähnlichkeitsfunktion die Summe $D_{xx}(k)$ der Betragsdifferenzen verwendet wird.

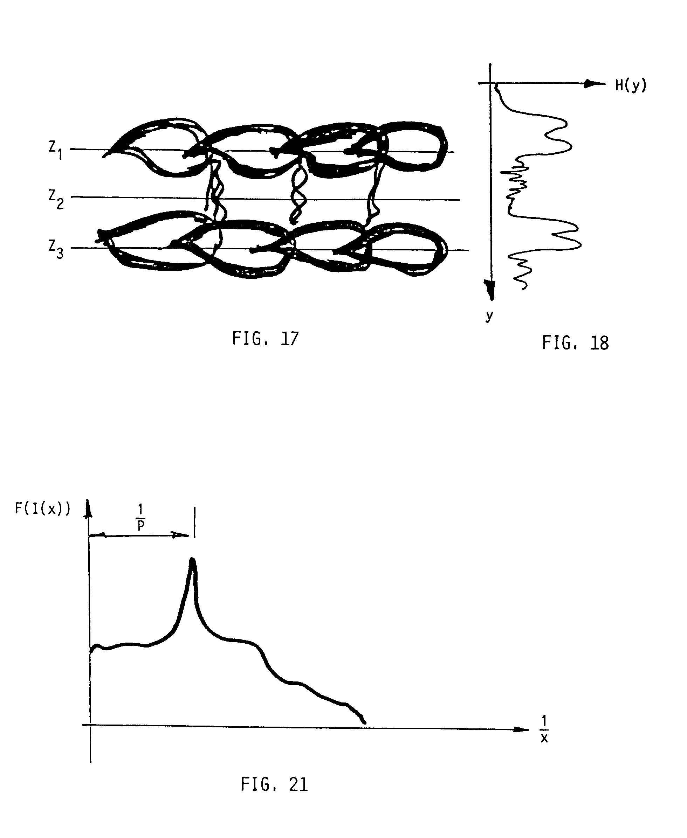

Zur Reduzierung des Hardware-Aufwands und des Zeitbedarfs bei der Berechnung und Auswertung der Ähnlichkeitsfunktion ist es vorteilhaft, wenn nur Bildzeilen ausgewertet werden, in denen die Maschenperiode besonders deutlich hervortritt. Dies sind insbesondere die Bildzeilen, die mittig durch die Maschenlöcher verlaufen, da die hieraus berechneten Ähnlichkeitsfunktionen besonders ausgeprägte Maxima und Minima aufzeigen. Dadurch kann gegebenenfalls die Notwendigkeit einer weiteren Mittelung entfallen. Beispielsweise ergeben bei dem Maschenbild von Fig. 17 die Bildzeilen $Z_1$ und $Z_3$ besonders deutliche Ähnlichkeitsfunktionen, während die Bildzeile $Z_2$ infolge des schwachen Kontrastes nur sehr undeutliche Ähnlichkeitsfunktionen liefern kann. Zur Ermittlung der am besten geeigneten Bildzeilen wird das Helligkeitsprofil $H(y)$ quer zur Auswertungsrichtung gebildet, wie es in Fig. 18 in räumlicher Beziehung zum Maschenbild von Fig. 17 dargestellt ist, und es werden diejenigen Bildzeilen ausgewertet, die im Profilgebirge die am stärksten ausgeprägten Helligkeitsunterschiede aufweisen. Die Bildung des Helligkeitsprofils $H(y)$ erfolgt bei der Anordnung von Fig. 1 in der Recheneinheit 11 aufgrund der im Bildsignalspeicher 7 bzw. 10 gespeicherten Bildsignale. Die Recheneinheit 11 wertet das Helligkeitsprofil $H(y)$ aus und bestimmt die Adressen der gespeicherten Bildsignale, die für die Berechnung der Ähnlichkeitsfunktion heranzuziehen sind. Durch diese automatische Auswahl besonders gut ausgeprägter Bildzeilen ist es möglich, bereits mit der Auswertung von sehr wenigen Bildzeilen zu genauen Meßergebnissen zu kommen.

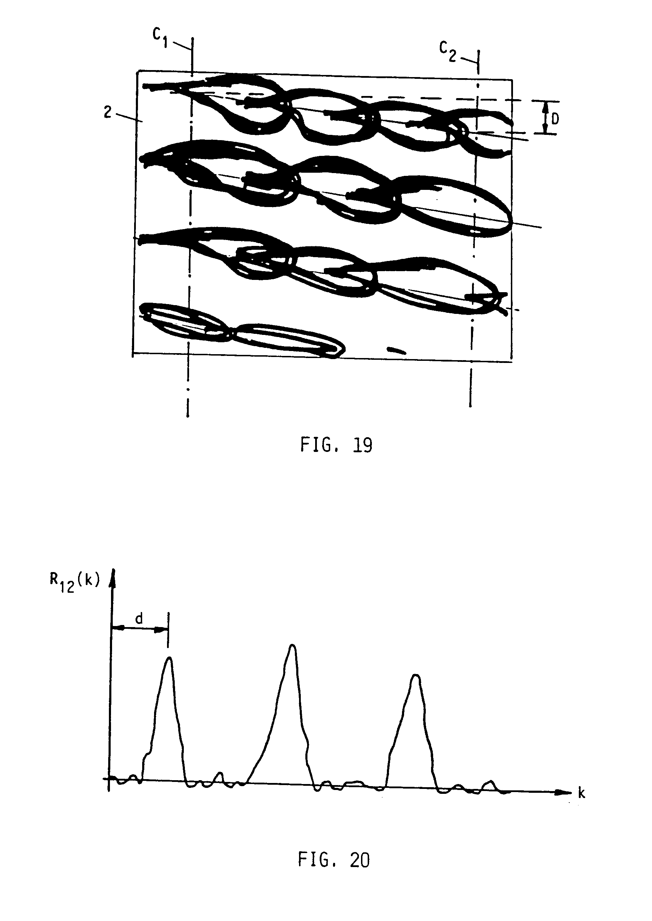

Oft läßt sich in der Praxis ein Schräglauf der Textiloberfläche im Bildfenster der Fernsehkamera nicht vermeiden, da insbesondere bei Strickwaren die genaue Stoffführung erhebliche technische Schwierigkeiten bereitet. Um dennoch zu einer verwertbaren eindimensionalen Ähnlichkeitsfunktion zu gelangen, ist es erforderlich, diese entlang der Schräglinie des Maschenverlaufs zu berechnen. Fig. 19 zeigt als Beispiel den von einer Fernsehkamera erfaßten Bildausschnitt der Textilbahn 2 mit schräg zur Zeilenrichtung verlaufenden Maschenlinien.

Die Bestimmung des Schräglaufs kann dadurch erfolgen, daß die Kreuzkorrelationsfunktion $R_{12}(k)$ oder eine entsprechende Ähnlichkeitsfunktion zwischen den Bildsignalen berechnet wird, die einerseits einer Bildspalte $C_1$ am linken Bildrand und andrerseits einer Bildspalte $C_2$ am rechten Bildrand entnommen werden. Die Kreuzkorrelationsfunktion ist definiert durch die Summe der paarweisen Produkte der Helligkeitswerte von Bildpunkten der einen Bildspalte $C_1$ mit den Helligkeitswerten von Bildpunkten der um k Bildpunkte verschobenen anderen Bildspalte $C_2$, wobei matürlich in der Spaltenrichtung der Bildpunktabstand $\Delta y$ durch den Zeilenabstand oder ein ganzzahliges Vielfaches des Zeilenabstandes gegeben ist. Die Berechnung der Kreuzkorrelationsfunktion $R_{12}(k)$ erfolgt wieder durch die Recheneinheit 11 aufgrund der in dem betreffenden Bildsignalspeicher 7 bzw. 10 gespeicherten Bildsignale. Ihr Verlauf ist in Fig. 20 dargestellt. Der Abstand d des ersten Maximums vom Ursprung entspricht (mit dem durch den Wert $k \cdot \Delta y$ bestimmten Umrechnungsfaktor) der Verschiebung D der Maschenmittenlinie zwischen den Bildspalten $C_1$ und $C_2$. Bei Kenntnis der Verschiebung D kann die Recheneinheit 11 die Adressen der gespeicherten Bildsignale berechnen, die einer entlang den schrägen Maschenmittenlinien verlaufenden Bildpunktreihe stammen. Dadurch wird das Meßproblem wieder auf die Berechnung einer eindimensionalen Ähnlichkeitsfunktion zurückgeführt. Die ausgewertete Bildpunktreihe fällt in diesem Fall nicht mit einer Bildzeile zusammen, sondern verläuft schräg über mehrere Bildzeilen.

Bei starkem Schräglauf kann es zweckmäßig sein, in der Recheneinheit 11 die zweidimensionale Autokorrelationsfunktion bzw. eine mit ihr verwandte zweidimensionale Ähnlichkeitsfunktion zu bilden. Die Maschenperiode wird dann aus dem erhaltenen zweidimensionalen Korrelationsgebirge durch die Linie der am stärksten ausgeprägten Gipfel- und Talfolgen bestimmt. Die Ermittlung zweidimensionaler Korrelationsfunktionen ist Stand der Technik und braucht hier nicht näher erläutert zu werden.

Für den Fachmann sind zahlreiche äquivalente Abwandlungen des beschriebenen Verfahrens und der zu seiner Durchführung angegebenen Anordnung offensichtlich. So wurde in der vorstehenden Beschreibung angenommen, daß die zur Gewinnung der Bildsignale verwendeten Bild/Signal-Wandler Fernsehkameras sind, die den Bildausschnitt in einem genormten Fernsehraster zeilenweise abtasten. Die Erfindung ist jedoch nicht auf die Verwendung bestimmter Bild/Signal-Wandler beschränkt. Anstelle von Fernsehkameras können insbesondere auch normfreie Matrix- oder Zeilensensoren verwendet werden, wie sie in verschiedenen Ausführungen im Handel erhältlich sind. Besonders gut geeignet sind beispielsweise CCD-Halbleiter-Matrix-Kameras mit elektronisch veränderbarer Integrationszeit, da deren Verwendung gegebenenfalls eine stroboskopische Beleuchtung zur Vermeidung von Bewegungsunschärfen bei bewegter Textilbahn erübrigt.

Die Verwendung anderer Bild/Signal-Wandler als Fernsehkameras ist insbesondere auch deshalb möglich, weil es nicht erforderlich ist, die schnelle Bildfolgefrequenz von Fernsehkameras auszunutzen. Einerseits ändert sich die Schrumpfung im Verlauf des Herstellungsprozessen nur verhältnismäßig langsam, so daß es genügt, die Messungen in Zeitabständen durchzuführen, die groß gegen die Bildperiode einer Fernsehkamera sind; andrerseits ist der Rechenaufwand für die Ermittlung und Auswertung der Ähnlichkeitsfunktionen so groß, daß er allenfalls mit sehr teuren Hochgeschwindigkeitsrechnern innerhalb der Bildperiode einer Fernsehkamera durchgeführt werden könnte. Bei einer praktischen Anwendung des zuvor beschriebenen Verfahrens erfolgen die Messungen fortlaufend in Zeitabständen von etwa 5 bis 10 Sekunden. Dies reicht für die Berechnung und Auswertung der Ähnlichkeitsfunktionen durch einen Computer mit einem 32 Bit-Mikroprozessor aus.

Um den Einfluß der Farbgebung des zu untersuchenden Materials auf das Meßergebnis zu verringern, ist es auch möglich, anstatt der Helligkeitsstruktur die periodischen Höhenunterschiede der Textilbahn auszuwerten. Dazu wird die Oberfläche der Textilbahn in einem so flachen Einfallswinkel beleuchtet, daß die periodischen leichten Höhenunterschiede der Maschen ein entsprechendes periodisches Schattenmuster erzeugen. Dieses Schattenmuster ist unabhängig von der Farbe der Textilware, so daß auch komplett schwarze Ware durch den Wechsel von Schattenwurf und beleuchtetem Maschengipfel ausgewertet werden kann.

Die mittlere Grundperiode des Maschenbildes läßt sich anstatt durch eine Ähnlichkeitsfunktion auch aus dem Maximum einer spektralen Transformation des Maschenbildes bestimmen. Dies kann durch die Verwendung der Fourier-Transformation oder einer mit ihr verwandten orthogonalen Transformationen, der Walsh-Transformation oder der Hadamard-Transformation geschehen. Da für zahlreiche dieser Transformationen schnelle Algorithmen bekannt sind (z.B. der Fast Fourier Transformation Algorithmus), kann durch deren Anwendung insbesondere bei der Berechnung durch Mikrocomputer eine Zeitersparnis erreicht werden. Die Maschengrundperiode wird in diesem Fall aus dem dominierenden Maximum des Spektrums gewonnen, wie in Fig. 21 für die Fourier-Transformierte $F(I(x))$ dargestellt ist. Da die Autokorrelationsfunktion und die Fourier-Transformation Transformationspaare darstellen, enthalten beide die gleiche Information, allerdings in einer unterschiedlichen Darstellung.

## Patentansprüche

1.  Verfahren zur kontinuierlichen berührungslosen Messung der Schrumpfung von Textilien (2) während des Herstellungsprozesses, dadurch gekennzeichnet, daß mit Hilfe eines Bild/Signal-Wandlers (3,4) das Bild eines Ausschnitts der Textiloberfläche jeweils vor und nach der Schrumpfung aufgenommen und in analoge elektrische Bildsignale umgewandelt wird, daß die analogen Bildsignale bildpunktweise digitalisiert und abgespeichert werden, daß aus den gespeicherten digitalen Bildsignalen die Periode der Textilstruktur in wenigstens einer Richtung vor und nach der Schrumpfung abgeleitet wird, und daß die durch die Schrumpfung bewirkte Längenänderung der Textilware (2) in der Auswertungsrichtung aus dem Verhältnis der beiden in dieser Richtung ermittelten Perioden berechnet wird.

2.  Verfahren nach Anspruch 1, dadurch gekennzeichnet, daß die Perioden der Textilstruktur in zwei zueinander senkrechten Auswertungsrichtungen ermittelt werden.

**3.** Verfahren nach Anspruch 2, dadurch gekennzeichnet, daß zur Messung der Schrumpfung einer kontinuierlich bewegten Textilbahn (2) die Perioden der Textilstruktur in der der Bewegungsrichtung entsprechenden Längsrichtung der Textilbahn (2) und in der senkrecht zur Bewegungsrichtung liegenden Querrichtung der Textilbahn (2) ermittelt werden.

**4.** Verfahren nach einem der vorhergehenden Ansprüche, dadurch gekennzeichnet, daß zur Ableitung der Perioden der Textilstruktur eine eindimensionale Ähnlichkeitsfunktion gespeicherter digitaler Bildsignale gebildet wird, die von wenigstens einer in der Auswertungsrichtung verlaufenden Bildpunktreihe stammen.

**5.** Verfahren nach Anspruch 4, dadurch gekennzeichnet, daß die eindimensionale Ähnlichkeitsfunktion die Autokorrelationsfunktion ist.

**6.** Verfahren nach Anspruch 4, dadurch gekennzeichnet, daß die eindimensionale Ähnlichkeitsfunktion die Summe der Betragsdifferenzen der digitalen Bildsignale der gegeneinander verschobenen Bildpunkte der gleichen Bildpunktreihe ist.

**7.** Verfahren nach einem der Ansprüche 4 bis 6, dadurch gekennzeichnet, daß für die Bildung der eindimensionalen Ähnlichkeitsfunktion Bildpunktreihen ausgewertet werden, in denen die Periodenstruktur infolge der am stärksten ausgeprägten Helligkeitsunterschiede deutlich hervortritt.

**8.** Verfahren nach Anspruch 7, dadurch gekennzeichnet, daß zur Auswahl der ausgewerteten Bildpunktreihen die Helligkeitsfunktion entlang wenigstens einer quer zur Auswertungsrichtung verlaufenden Bildpunktreihe gebildet wird.

**9.** Verfahren nach Anspruch 7 oder 8, dadurch gekennzeichnet, daß zur Bestimmung der Richtung der für die Bildung der eindimensionalen Ähnlichkeitsfunktion auszuwertenden Bildpunktreihen die Kreuzkorrelationsfunktion zwischen den Bildsignalen gebildet wird, die von zwei quer zur Auswertungsrichtung verlaufenden, im Abstand voneinander liegenden Bildpunktreihen stammen.

**10.** Verfahren nach einem der Ansprüche 4 bis 9, dadurch gekennzeichnet, daß zur Bildung der eindimensionalen Ähnlichkeitsfunktion die gespeicherten digitalen Bildsignale einer Bildpunktreihe von M Bildpunkten mit den gespeicherten digitalen Bildsignalen eines um k Bildpunkte fortlaufend verschobenen Auszugs von M-k Bildpunkten aus der gleichen Bildpunktreihe verglichen werden und die Verschiebung k über den Wert M/2 hinaus erhöht wird.

**11.** Verfahren nach einem der Ansprüche 4 bis 10, dadurch gekennzeichnet, daß für die Ähnlichkeitsfunktion ein Gütekriterium vorgegeben wird und daß nur solche Ausschnitte der Ähnlichkeitsfunktion für die Ermittlung der Periode der Textilstruktur ausgewertet werden, die dieses Gütekriterium erfüllen.

**12.** Verfahren nach Anspruch 1, dadurch gekennzeichnet, daß für Maxima und/oder Minima der Ähnlichkeitsfunktion ein Schwellwert vorgegeben wird und daß nur solche Maxima und/oder Minima der Ähnlichkeitsfunktion ausgewertet werden, die den vorgegebenen Schwellwert über- bzw. unterschreiten.

**13.** Verfahren nach einem der Ansprüche 4 bis 12, dadurch gekennzeichnet, daß die Periode der Textilstruktur durch Bildung des Mittelwerts mehrerer aus der gleichen Ähnlichkeitsfunktion gewonnener Perioden abgeleitet wird.

**14.** Verfahren nach Anspruch 13, dadurch gekennzeichnet, daß die aus der gleichen Ähnlichkeitsfunktion gewonnenen Perioden zur Bildung des Mittelwerts gewichtet werden.

**15.** Verfahren nach einem der Ansprüche 4 bis 14, dadurch gekennzeichnet, daß die Ähnlichkeitsfunktion vor ihrer Auswertung digital geglättet wird.

**16.** Verfahren nach einem der Ansprüche 1 bis 3, dadurch gekennzeichnet, daß zur Ableitung der Perioden der Textilstruktur eine zweidimensionale Ähnlichkeitsfunktion gespeicherter digitaler Bildsignale gebildet wird.

**17.** Verfahren nach einem der Ansprüche 1 bis 3, dadurch gekennzeichnet, daß die Perioden der Textilstruktur aus einer spektralen Transformation des durch die gespeicherten digitalen Bildsignale dargestellten Maschenbildes abgeleitet werden.

**18.** Verfahren nach einem der Ansprüche 1 bis 17, dadurch gekennzeichnet, daß das vom Bild/Signal-Wandler (3,4) abgegebene analoge Bildsignal vor der Digitalisierung hochpaßgefiltert wird.

**19.** Verfahren nach einem der Ansprüche 1 bis 18, dadurch gekennzeichnet, daß das analoge Bildsignal zur Digitalisierung durch Vergleich mit einem Schwellenwert binarisiert wird.

**20.** Verfahren nach einem der Ansprüche 1 bis 19, dadurch gekennzeichnet, daS die Textiloberfläche synchron zum Bildwechsel des Bild/Signal-Wandlers (3,4) stroboskopisch beleuchtet wird.

**21.** Verfahren nach einem der Ansprüche 1 bis 20, dadurch gekennzeichnet, daß die Textiloberfläche zur Erzeugung von Schattenwürfen der Erhöhungen mit Licht beleuchtet wird, das unter einem sehr kleinen Winkel zur Oberfläche einfällt.

**22.** Anordnung zur Durchführung des Verfahrens nach einem der vorhergehenden Ansprüche, gekennzeichnet durch einen Bild/Signal-Wandler (3), der das Bild eines Ausschnitts der Textiloberfläche vor der Schrumpfung aufnimmt und in ein analoges elektrisches Bildsignal umwandelt, einen Bild/Signal-Wandler (4), der das Bild eines Ausschnitts der Textiloberfläche nach der Schrumpfung aufnimmt und in ein analoges elektrisches Bildsignal umwandelt, einen jedem Bild/Signal-Wandler (3,4) nachgeschalteten Analog/Digital-Umsetzer (6,9) zur Digitalisierung der analogen Bildsignale, wenigstens einen Bildsignalspeicher (7,10) zur Speicherung der von den Analog/Digital-Umsetzern (6,9) gelieferten digitalen Bildsignale und durch eine Recheneinheit (11) zur Ableitung der Periode der Textilstruktur aus den gespeicherten digitalen Bildsignalen.

**23.** Anordnung nach Anspruch 22, dadurch gekennzeichnet, daß jeder Bild/Signal-Wandler eine Fernsehkamera (3,4) ist.

**24.** Anordnung nach Anspruch 23 zur Messung der Schrumpfung einer kontinuierlich bewegten Textilbahn, dadurch gekennzeichnet, daß die Fernsehkamera (3, 4) so ausgerichtet ist, daß die Zeilenabtastrichtung parallel zur Bewegungsrichtung der Textilbahn (2) verläuft.

**25.** Anordnung nach Anspruch 22, dadurch gekennzeichnet, daß jeder Bild/Signal-Wandler (3,4) ein Matrix- oder Zeilensensor ist.

**26.** Anordnung nach Anspruch 25, dadurch gekennzeichnet, daß jeder Bild/Signal-Wandler (3,4) eine CCD-Halbleiter-Matrix-Kamera ist.

**27.** Anordnung nach einem der Ansprüche 22 bis 25, gekennzeichnet durch ein Stroboskop, das die Textiloberfläche synchron mit der Bildwechselfrequenz des Bild/Signal-Wandlers (3,4) beleuchtet.

**28.** Anordnung nach Anspruch 27, dadurch gekennzeichnet, daß bei Verwendung von Infrarot-empfindlichen Bild/Signal-Wandlern (3,4) das Stroboskop ein Infrarot-Blitzgerät ist.

**29.** Anordnung nach einem der Ansprüche 22 bis 28, dadurch gekennzeichnet, daß zwischen jedem Bild/Signal-Wandler (3,4) und dem zugeordneten Analog/Digital-Umsetzer (6,9) eine Analogsignalverarbeitungsschaltung (5,8) vorgesehen ist.

**30.** Anordnung nach Anspruch 29, dadurch gekennzeichnet, daß jede Analogsignalverarbeitungsschaltung (5,8) ein Hochpaßfilter enthält.

## Claims

**1.** Method for the continuous contactless measurement of the shrinkage of textiles (2) during the production process, characterized in that with the aid of an image/signal transducer (3, 4) the image of a portion of the textile surface is picked up both before and after the shrinkage and converted to analog

EP 0 160 895 B1

electrical image signals; that the analog electrical image signals are digitized and stored in image point manner; that from the stored digital image signals the period of the textile structure in at least one direction before and after the shrinkage is derived; and that the change of length of the textile material (2) effected by the shrinkage is calculated in each evaluation direction from the ratio of the two periods determined in this direction.

2. Method according to claim 1, characterized in that the periods of the textile structure are determined in two evaluation directions perpendicular to each other.

3. Method according to claim 2, characterized in that for measuring the shrinkage of a continuously moving textile web (2) the periods of the textile structure in the longitudinal direction of the textile web (2) corresponding to the direction of movement and in the transverse direction of the textile web (2) lying perpendicular to the direction of movement are determined.

4. Method according to anyone of the preceding claims, characterized in that for deriving the periods of the textile structure a unidimensional similarity function of stored digital image signals is formed which originate from at least one image point row extending in the evaluation direction.

5. Method according to claim 4, characterized in that the unidimensional similarity function is the autocorrelation function.

6. Method according to claim 4, characterized in that the unidimensional similarity function is the sum of the quantity differences of the digital image signals of the mutually displaced image points of the same image point row.

7. Method according to anyone of claims 4 to 6, characterized in that for the formation of the unidimensional similarity function image point rows are evaluated in which the period structure is clearly apparent, as a consequence of the strongest marked brightness differences.

8. Method according to claim 7, characterized in that for selecting the evaluated image point rows the brightness function is formed along at least one image point row extending transversely of the evaluation direction.

9. Method according to claim 7 or 8, characterized in that for determining the direction of the image point rows to be evaluated for forming the unidimensional similarity function the cross-correlation function is formed between the image signals which originate from two image point rows extending transversely of the evaluation direction and spaced apart from each other.

10. Method according to anyone of claims 4 to 9, characterized in that for forming the unidimensional similarity function the stored digital image signals of an image point row of M image points are compared with the stored digital image signals of an extract of M-k image points from the same point row displaced continuously through k image points and the displacement k is increased beyond the value M/2.

11. Method according to anyone of claims 4 to 10, characterized in that a quality criterion is predetermined for the similarity function; and in that only such portions of the similarity function which fulfil this predetermined quality criterion are evaluated for determining the period of the textile structure.

12. Method according to claim 1, characterized in that a threshold value is predetermined for the maxima and/or minima of the similarity function; and that only those maxima and/or minima of the similarity function which exceed or are below this predetermined threshold value are evaluated.

13. Method according to anyone of claims 4 to 12, characterized in that the period of the textile structure is derived by forming the mean value of a plurality of periods obtained from the same similarity function.

14. Method according to claim 13, characterized in that the periods obtained from the same similarity function are weighed to form the mean value.

13

**15.** Method according to anyone of claims 4 to 14, characterized in that the similarity function is digitally smoothed prior to its evaluation.

**16.** Method according to anyone of claims 1 to 3, characterized in that for deriving the periods of the textile structure a two-dimensional similarity function of stored digital image signals is formed.

**17.** Method according to anyone of claims 1 to 3, characterized in that the periods of the textile structure are derived from a spectral transformation of the stitch image represented by the stored digital image signals.

**18.** Method according to anyone of claims 1 to 17, characterized in that the analog image signal furnished by the image/signal transducer (3, 4) is high-pass filtered prior to digitizing.

**19.** Method according to anyone of claims 1 to 18, characterized in that the analog image signal is binarized for digitizing by comparison with a threshold value.

**20.** Method according to anyone of claims 2 to 19, characterized in that the textile surface is illuminated synchronously with the frame change of the image/signal transducer (3, 4) in stroboscopic manner.

**21.** Method according to anyone of claims 1 to 20, characterized in that the textile surface is illuminated for generating shadows of the protuberances with light incident at the surface at a very small angle.

**22.** Arrangement for carrying out the method of anyone of the preceding claims, characterized by comprising an image/signal transducer (3) which picks up the image of a portion of the textile structure before shrinkage and converts said image to an analog electrical image signal, an image/signal transducer (4) which picks up the image of a portion of the textile surface after the shrinkage and converts said image to an analog electrical image signal, an analog/digital converter (6, 9) following each image/signal transducer (3, 4) for digitizing the analog image signals, at least one image signal memory (7, 10) for storing the digital image signals furnished by the analog/digital converter (6, 9) and a calculating unit (11) for deriving the period of the textile structure from the stored digital image signals.

**23.** Arrangement according to claim 22, characterized in that each image/signal transducer is a television camera (3, 4).

**24.** Arrangement according to claim 23 for measuring the shrinkage of a continuously moving textile web, characterized in that the television camera (3, 4) is so aligned that the line scan direction runs parallel to the direction of movement of the textile web (2).

**25.** Arrangement according to claim 22, characterized in that each image/signal transducer (3, 4) is a matrix or line sensor.

**26.** Arrangement according to claim 25, characterized in that each image/signal transducer (3, 4) is a CCD semiconductor matrix camera.

**27.** Arrangement according to anyone of claims 22 to 25, characterized by a stroboscope which illuminates the textile surface synchronously with the frame frequency of the image/signal transducer (3, 4).

**28.** Arrangement according to claim 27, characterized in that when using infrared-sensitive image/signal transducers (3, 4) the stroboscope is an infrared flash device.

**29.** Arrangement according to claim 22, characterized in that between each image/signal transducer (3, 4) and the associated analog/digital converter (6, 9), an analog signal processing circuit (5, 8) is provided.

**30.** Arrangement according to claim 29, characterized in that each analog signal processing circuit (5, 8) contains a high-pass filter.

**Revendications**

1. Procédé pour la mesure continue sans contact du rétrécissement de textiles (2) au cours du processus de fabrication, caractérisé en ce que, à l'aide d'un convertisseur image/signal (3, 4), on saisit l'image d'une partie de la surface du textile à la fois avant et après le rétrécissement et on la convertit en signaux d'image électriques analogiques, en ce que l'on numérise les signaux d'image électriques analogiques et on les stocke suivant les points d'image, en ce que l'on dérive à partir des signaux d'image numérisés stockés la période de la structure du textile dans au moins une direction avant et après le rétrécissement, et en ce que le changement de longueur du matériau textile (2) provoqué par le rétrécissement est calculé dans chaque direction d'évaluation à partir du rapport des deux périodes déterminées dans cette direction.

2. Procédé selon la revendication 1, caractérisé en ce que les périodes de la structure du textile sont déterminées dans deux directions d'évaluation perpendiculaires l'une à l'autre.

3. Procédé selon la revendication 2, caractérisé en ce que pour mesurer le rétrécissement d'une bande textile (2) en déplacement continu, on détermine les périodes de la structure du textile dans la direction longitudinale de la bande textile (2) qui correspond à la direction de déplacement et dans la direction transversale de la bande textile (2) qui s'étend perpendiculairement à la direction de déplacement.

4. Procédé selon l'une quelconque des revendications précédentes, caractérisé en ce que pour dériver les périodes de la structure textile on forme une fonction de similarité unidimensionnelle des signaux d'image numérisés stockés qui proviennent d'au moins une rangée de points d'image qui s'étend dans la direction d'évaluation.

5. Procédé selon la revendication 4, caractérisé en ce que la fonction de similarité unidimensionnelle est la fonction d'autocorrélation.

6. Procédé selon la revendication 4, caractérisé en ce que la fonction de similarité unidimensionnelle est la somme des différences quantitatives des signaux d'image numérisés des points d'image mutuellement décalés de la même rangée de points d'image.

7. Procédé selon l'une quelconque des revendications 4 à 6, caractérisé en ce que pour la formation de la fonction de similarité unidimensionnelle, on évalue des rangées de points dans lesquelles la structure de période est clairement apparente en conséquence des différences de luminosité les plus marquées.

8. Procédé selon la revendication 7, caractérisé en ce que pour choisir les rangées de points d'image évalués, on forme la fonction de luminosité le long d'au moins une rangée de points d'image qui s'étend transversalement à la direction d'évaluation.

9. Procédé selon l'une ou l'autre des revendications 7 et 8, caractérisé en ce que pour déterminer la direction des rangées de points d'image à évaluer pour former la fonction de similarité unidimensionnelle, on forme la fonction de corrélation croisée entre les signaux d'image qui proviennent de deux rangées de points d'image qui s'étendent transversalement à la direction d'évaluation et écartées l'une de l'autre.

10. Procédé selon l'une quelconque des revendications 4 à 9, caractérisé en ce que pour former la fonction de similarité unidimensionnelle on compare les signaux d'image numérisés stockés d'une rangée de M points d'image avec les signaux d'image numérisés stockés de M-k points d'image de la même rangée de points d'image, déplacés de façon continue de k points d'image et on augmente le déplacement k au-delà de la valeur M/2.

11. Procédé selon l'une quelconque des revendications 4 à 10, caractérisé en ce que l'on prédétermine un critère de qualité pour la fonction de similarité et que l'on évalue uniquement celles des parties de la fonction de similarité qui remplissent ce critère de qualité prédéterminé pour déterminer la période de la structure du textile.

12. Procédé selon la revendication 1, caractérisé en ce que l'on prédétermine une valeur de seuil pour les maxima et/ou les minima de la fonction de similarité, et en ce que l'on évalue ceux des maxima et/ou

EP 0 160 895 B1

minima de la fonction de similarité qui dépassent ou sont inférieurs à cette valeur de seuil prédéterminée.

**13.** Procédé selon l'une quelconque des revendications 4 à 12, caractérisé en ce que l'on dérive la période de la structure du textile en formant la valeur moyenne d'une pluralité de périodes obtenues avec la même fonction de similarité.

**14.** Procédé selon la revendication 13, caractérisé en ce que les périodes obtenues avec la même fonction de similarité sont pondérées pour former la valeur moyenne.

**15.** Procédé selon l'une quelconque des revendications 4 à 14, caractérisé en ce que la fonction de similarité est lissée de manière numérique avant d'être évaluée.

**16.** Procédé selon l'une quelconque des revendications 1 à 3, caractérisé en ce que pour dériver les périodes de la structure du textile on forme une fonction de similarité bi-dimensionnelle des signaux d'image numérisés stockés.

**17.** Procédé selon l'une quelconque des revendications 1 à 3, caractérisé en ce que les périodes de la structure du textile sont dérivées à partir d'une transformation spectrale de l'image de maille représentée par les signaux d'image numérisés stockés.

**18.** Procédé selon l'une quelconque des revendications 1 à 17, caractérisé en ce que le signal d'image analogique fourni par le convertisseur image/signal (3, 4) est passé dans un filtre passe-haut avant d'être numérisé.

**19.** Procédé selon l'une quelconque des revendications 1 à 18, caractérisé en ce que le signal d'image analogique est mis sous forme binaire pour la numérisation par comparaison avec une valeur de seuil.

**20.** Procédé selon l'une quelconque des revendications 1 à 19, caractérisé en ce que la surface du textile est illuminée de manière stroboscopique en synchronisme avec le changement de trame du convertisseur image/signal (3, 4).

**21.** Procédé selon l'une quelconque des revendications 1 à 20, caractérisé en ce que la surface du textile est illuminée afin de produire des ombres des reliefs avec une lumière incidente à la surface sous un angle très faible.

**22.** Agencement pour la mise en oeuvre du procédé selon l'une quelconque des revendications précédentes, caractérisé en ce qu'il comprend un convertisseur image/signal (3) qui saisit l'image d'une partie de la surface du textile avant rétrécissement et convertit cette image en un signal d'image électrique analogique, un convertisseur image/signal (4) qui saisit l'image d'une partie de la surface du textile après rétrécissement et convertit cette image en un signal d'image électrique analogique, un convertisseur analogique/numérique (6, 9) à la suite de chaque convertisseur image/signal (3, 4) pour numériser les signaux d'image analogiques, au moins une mémoire (7, 10) de signal d'image pour stocker les signaux d'image numérisés fournis par les convertisseurs analogiques/numériques (6, 9) et une unité de calcul (11) pour dériver la période de la structure du textile à partir des signaux d'image numérisés stockés.

**23.** Agencement selon la revendication 22, caractérisé en ce que chaque convertisseur image/signal est une caméra de télévision (3, 4).

**24.** Agencement selon la revendication 23, destiné à mesurer le rétrécissement d'une bande de textile en déplacement continu, caractérisé en ce que la caméra de télévision (3, 4) est alignée de telle manière que la direction du balayage de ligne court parallèlement à la direction de déplacement de la bande textile (2).

**25.** Agencement selon la revendication 22, caractérisé en ce que chaque convertisseur image/signal (3, 4) est un détecteur matriciel ou linéaire.

16

**26.** Agencement selon la revendication 25, caractérisé en ce que chaque convertisseur image/signal (3, 4) est une caméra matricielle à semi-conducteur à couplage de charge CCD.

**27.** Agencement selon l'une quelconque des revendications 22 à 25, caractérisé en ce qu'il comprend un stroboscope qui illumine la surface du textile en synchronisme avec la fréquence de trame du convertisseur image/signal (3, 4).

**28.** Agencement selon la revendication 27, caractérisé en ce que, lorsque l'on utilise des convertisseurs image/signal (3, 4) sensibles aux infra-rouges, le stroboscope est un dispositif à éclairs infra-rouges.

**29.** Agencement selon la revendication 22, caractérisé en ce qu'il est prévu un circuit de traitement de signal analogique (5, 8) entre chaque convertisseur image/signal (3, 4) et le convertisseur analogique/numérique associé (6, 9).

**30.** Agencement selon la revendication 29, caractérisé en ce que chaque circuit de traitement de signal analogique (5, 8) contient un filtre passe-haut.

FIG. 1

FIG. 2

FIG. 5

Bildpunkt
Nr. 1 2 3 4 5 ...          M
i = 0 1 2 3 4 ...  Δx   M-1          x = i·Δx

FIG. 6

FIG. 3

FIG. 4

FIG. 7

FIG. 8

FIG. 9

EP 0 160 895 B1

FIG. 10

EP 0 160 895 B1

FIG. 11

FIG. 12

25

FIG. 13

FIG. 14

FIG. 15

FIG. 16

FIG. 17

FIG. 18

FIG. 21

FIG. 19

FIG. 20